# EUROPÄISCHE PATENTSCHRIFT

(11) **EP 0 979 638 B1**
(45) Veröffentlichungstag und Bekanntmachung des Hinweises auf die Patenterteilung: **04.12.2002**
(21) Anmeldenummer: 99115874.2
(22) Anmeldetag: 12.08.1999
(51) Int. Cl.: A61F 2/44, A61B 17/80

(54) **Wirbelsäulen-Implantat**
Spinal implant
Implant vertébral

(30) Priorität: 13.08.1998 DE 19836643
(43) Veröffentlichungstag der Anmeldung: 16.02.2000
(73) Patentinhaber: Dirik, Erkan, 70376 Stuttgart (DE)
(72) Erfinder: Dirik, Erkan, 70376 Stuttgart (DE)
(74) Vertreter: Lucht, Silvia

(56) Entgegenhaltungen:
- WO-A-97/15248
- DE-U- 9 216 092
- DE-U- 29 511 146
- DE-U- 29 623 361
- DE-U- 29 703 043
- FR-A- 2 727 005
- FR-A- 2 742 653
- FR-A- 2 747 034

## Beschreibung

### Stand der Technik

Die Erfindung geht aus von einem Wirbelsäulen-Implantat nach dem Oberbegriff des Anspruchs 1.

Bandscheibenvorfälle, Osteochondrose und Spondylosis deformans sind häufig Ursachen für starke Schmerzen und Bewegungseinschränkungen eines oder mehrerer Segmente der Wirbelsäule mit der Folge einer reflektorischen Muskelverspannung, einer Fehlhaltung der Wirbelsäule oder weiteren Begleiterscheinungen. Falls radikuläre Beschwerden mit neurologischen Ausfällen oder Marksymptomatik auftreten, ist eine operative Behandlung notwendig.

Aus dem Stand der Technik ist bekannt, zur Behandlung dem Patienten beispielsweise aus dem Beckenkamm ein Knochenstück zu entnehmen und dieses anstelle der defekten Bandscheibe zwischen die Wirbel einzusetzen. Als nachteilig erweist sich hierbei, daß zwei Operationen mit jeweils relativ langer Operationszeit notwendig sind. Dabei können außerdem Infektionen, Hämatome oder Verletzungen von Nerven auftreten. Außerdem besteht die Gefahr einer Verletzung der im Becken verlaufenden Nerven, insbesondere des Nervus cutaneus formalis lateralis, des Nervus ilioinguinalis, des Nervus genitofemoralis und des Nervus iliohypogastricus. Im übrigen erhöht sich die Gefahr eines Bruches an der Stelle, an der das Knochenstück entnommen wurde.

Darüber hinaus ist eine Behandlung bekannt, bei der zunächst die defekte Bandscheibe entfernt und der zwischen den Wirbeln verbleibende Zwischenraum mit einer zunächst flüssigen Masse aufgefüllt wird. Die flüssige Masse erstarrt und führt zu einer Fusion mit den benachnarten Wirbeln. Als nachteilig erweist sich hierbei, daß sich die flüssige Masse zwischen den Wirbeln unkontrolliert ausbreiten kann. Dabei kann nicht verhindert werden, daß die Masse in Richtung des in den Wirbeln verlaufenden Nervenkanals fließt und im erstarrten Zustand einen Dorn bildet. Sofern dieser auf die Nerven drückt, hat der Patient auch nach der Operation Beschwerden und Schmerzen.

Aus dem Stand der Technik ist außerdem als Stützmittel für die Korrektur oder die Stabilisierung der Wirbelsäule eine an mindestens zwei benachbarten Wirbeln bzw. Wirbelkörpern anbringbare längliche Platte aus verformbarem körperverträglichen Material bekannt (G 91 14 118). Diese Platte wird mit Hilfe von Knochenschrauben an den Wirbeln befestigt. Sie dient jedoch in erster Linie der Stabilisierung oder Stützung der Wirbelsäule und kann zur Behandlung beispielsweise von Bandscheibenvorfällen nicht verwendet werden.

Aus der DE 297 03 043 U1 ist ein Wirbelsäulen-Implantat zur Stabilisierung der Wirbelsäule, insbesondere der Halswirbelsäule bekannt. Das Implantat besteht im wesentlichen aus einem flachen, zwischen zwei Wirbel einsetzbaren Körper, dessen Dicke ungefähr dem Abstand der Wirbel entspricht. Zur Aufnahme von Knochensubstanz sind in dem Körper Durchbrüche vorgesehen. An den den Wirbeln zugewandten Seiten des Körpers sind sägezahnförmige Verankerungselemente mit einer Spitze vorgesehen.

Aus der DE 43 27 054 C1 ist ein ventrales Zwischenwirbelimplantat mit mindestens einem aus der Anlagefläche des Implantats an dem Wirbelkörper herausragenden Ankerbolzen bekannt. Durch ein Getriebe in dem Implantat kann der Ankerbolzen ausgefahren werden. Um beim Ausfahren in den Wirbelkörper eindringen zu können, weisen die dem Wirbelkörper zugewandten Enden der Ankerbolzen eine Spitze auf. Durchbrüche ermöglichen das Einwachsen von Knochensubstanz in das Implantat.

### Die Erfindung und ihre Vorteile

Demgegenüber hat das Wirbelsäulen-Implantat mit den kennzeichnenden Merkmalen des Anspruchs 1 den Vorteil, daß ein flacher zwischen zwei Wirbeln einsetzbarer Körper vorgesehen ist, dessen Dicke ungefähr dem Abstand der Wirbel entspricht. In dem Körper sind mehrere durchgängige, nach außen offene Kanäle vorgesehen. Darüber hinaus ist der flache Körper an den den Wirbeln zugewandten Seiten mit mindestens einer halbkugelförmigen Erhebung ausgestattet, die ein Verbinden des Implantats mit den Wirbeln ermöglicht. Das Wirbelsäulen-Implantat kann mit Hilfe mikrochirurgischer Technik in die Wirbelsäule eingesetzt werden. Hierzu wird zunächst die defekte Bandscheibe vollständig entfernt oder die die neurologische Symptomatik verursachende degenerative Knochenveränderung abgetragen. Um das Knochenwachstum anzuregen, wird die Oberfläche der Wirbel aufgerauht. Anschließend wird das Wirbelsäulen-Implantat zwischen die Wirbel eingesetzt und über die noppenartigen Erhebungen fixiert. Die in dem Implantat vorgesehenen Kanäle bieten Spongiosa-Füllmöglichkeiten. Der Knochen wächst in die Kanäle hinein und führt damit zu einer optimalen und schnellen Fusion zwischen dem Implantat und den benachbarten Wirbeln. Die Kanäle können in dem Implantat sowohl in einer als auch in zwei oder drei Richtungen vorgesehen sein. Die physiologische Lordose, insbesondere im Halswirbelbereich, wird durch das Wirbelsäulen-Implantat erhalten. Die Patienten können ab dem ersten postoperativen Tag voll mobilisiert werden. Der Aufenthalt des Patienten im Krankenhaus verkürzt sich gegenüber bekannten Behandlungsmethoden beachtlich.

Das Wirbelsäulen-Implantat wird auf der ventralen Seite der Wirbelsäule eingesetzt. Dank des mit Hilfe mikrochirurgischer Methoden einsetzbaren erfindungsgemäßen Wirbelsäulen-Implantats ist die Narkosezeit wesentlich kürzer als bei bekannten Behandlungsmethoden und die Gefahr von Infektionen und Hämatomen nahezu beseitigt. Darüber hinaus ist lediglich eine Operation zur Behandlung notwendig.

Das erfindungsgemäße Wirbelsäulen-Implantat eignet sich insbesondere zum Einsatz in die Halswirbelsäule. Es kann jedoch auch in den übrigen Bereichen der Wirbelsäule eingesetzt werden.

Durch den Einsatz des Wirbelsäulen-Implantats wird der Patient hinsichtlich seiner Beweglichkeit nicht eingeschränkt. Es können auch bis zu drei Implantate zwischen benachbarten Wirbeln eingesetzt werden.

Die halbkugelförmigen Erhebungen sind starr mit dem flachen Körper des Implantats verbunden. Beim Einsetzen des Implantats in die Wirbelsäule wird in die Wirbel eine Vertiefung eingebracht, beispielsweise gebohrt, in welche die Erhebungen im eingesetzten Zustand eingreifen können. Durch den zwischen den Wirbeln herrschenden Druck wird das Implantat zwischen die Wirbel eingepreßt und dank der Erhebung in seiner Position fixiert. Das Verwachsen der Knochen mit dem Implantat führt zu einer zusätzlichen Stabilisierung.

Nach einer vorteilhaften Ausgestaltung der Erfindung ist als zusätzliche Befestigungsvorrichtung eine mit dem flachen Körper und mit mindestens zwei Wirbeln verbindbare Platte vorgesehen. Diese wird über Knochenschrauben mit den Wirbeln und über eine weitere Schraube mit dem flachen Körper verschraubt. Die Platte führt im Vergleich zu den an dem Implantat vorgesehenen in den Knochen eingreifenden Erhebungen zu einer zusätzlichen Stabilisierung und Stützung der Wirbelsäule.

Nach einer weiteren vorteilhaften Ausgestaltung der Erfindung sind die Öffnungen der Kanäle an den den Wirbeln zugewandten Seiten des Körpers vorgesehen. Die Kanäle weisen einen länglichen Querschnitt auf und können mit kleinen Knochenteilen des behandelten Patienten befüllt werden. Diese kleinen Knochenteile stammen von den Wirbeln, zwischen die das Implantat eingesetzt werden soll. Um das Knochenwachstum anzuregen, werden die Oberflächen der Wirbel auf den dem Imlantat zugewandten Seiten aufgeraut. Außerdem werden für die noppenartigen Befestigungsvorrichtungen kleine Löcher in die Wirbel gebohrt. Die bei diesen Behandlungen anfallenden Knochenteile werden gesammelt und vor dem Einsetzen des Implants in die Kanäle gepreßt. Die Wirbel verbinden sich mit der Knochensubstanz in den Kanälen, so daß eine besonders rasche Fusion zwischen dem Implantat und den benachbarten Wirbeln stattfindet.

Nach einer weiteren vorteilhaften Ausgestaltung der Erfindung ist an dem Körper an der im eingesetzten Zustand dem Nervenkanal der Wirbelsäule abgewandten Seite eine Gewindebohrung zum Einsetzen eines Werkzeugs vorgesehen. So kann beispielsweise eine Gewindestange in die Bohrung eingeschraubt werden. Diese ermöglicht ein exaktes Positionieren des Implantats zwischen den beiden Wirbeln. Befindet sich das Implantat an der vorgesehenen Stelle zwischen den Wirbeln und ist das Implantat über die Befestigungsvorrichtung zwischen den Wirbeln fixiert, dann kann die Gewindestange wieder entfernt werden, ohne daß das Implantat dabei seine Position verändert.

Nach einer weiteren vorteilhaften Ausgestaltung der Erfindung ist die den Wirbeln im eingesetzten Zustand zugewandte Fläche des Körpers kleiner als die Ausdehnung der Wirbel in der zur Wirbelsäule senkrechten Ebene. Dies führt dazu, daß das Wirbelsäulen-Implantat nicht aus der Wirbelsäule seitlich herausragt und bei entsprechendem Knochenwachstum vollständig mit Knochen umwachsen werden kann. Dabei können keine Ausbuchtungen entstehen, die die Umgebung der Wirbelsäule oder die in den Wibeln verlaufenden Nerven beeinträchtigen könnten. Die Fusion aus Wirbeln und Wirbelsäulen-Implantat zeichnet sich außerdem durch eine hohe Stabilität aus.

Nach einer weiteren vorteilhaften Ausgestaltung der Erfindung ist an dem Körper der im eingesetzten Zustand dem Nervenkanal der Wirbelsäule zugewandten Seite eine Einbuchtung vorgesehen. Auf diese Weise kann eine Beeinträchtigung der Nerven im Nervenkanal verhindert werden.

Nach einer weiteren vorteilhaften Ausgestaltung der Erfindung besteht der Körper aus Polyethylen, insbesondere aus gepreßtem ultrahochmolekularem Niederdruck-Polyethylen. Dieses Material hat den Vorteil, daß es resistent gegen sämtliche Körpersäfte, verschleißfest, stoßabsorbierend und körperverträglich ist. Außerdem kann es als hochreiner Rohstoff zur Verfügung gestellt werden und weist ein geringes Gewicht auf. Eine Sterilisation des Materials ist beispielsweise mit Gammastrahlen oder Ethylenoxyd möglich. Darüber hinaus beeinflußt das Wirbelsäulen-Implantat aus Polyethylen nicht die Untersuchungen mittels Kernspintomographie oder Computertomographie. Außerdem ist es leicht zu bearbeiten, was die Herstellung des Wirbelsäulen-Implantats vereinfacht.

Nach einer weiteren vorteilhaften Ausgestaltung der Erfindung besteht das Wirbelsäulen-Implantat aus Titan. Dieses Material ist ebenfalls resistent gegen Körpersäfte, verschleißfest und körperverträglich.

Weitere Vorteile und vorteilhafte Ausgestaltungen der Erfindung sind der nachfolgenden Beschreibung, der Zeichnung und den Ansprüchen entnehmbar.

### Zeichnung

In der Zeichnung ist ein Ausführungsbeispiel der Erfindung dargestellt und im folgenden näher beschrieben. Es zeigen:
- Fig.1: Wirbelsäulen-Implantat in perspektivischer Ansicht
- Fig. 2: Wirbelsäulen-Implantat aus Fig. 1 in einer Ansicht von oben,
- Fig. 3: Wirbelsäulen-Implantat aus Fig. 1 in einer Ansicht von der Seite,
- Fig. 4: Schnitt durch eine Anordnung aus zwei Wirbeln und einem Wirbelsäulen-Implantat,
- Fig. 5: Anordnung aus Fig. 4 in perspektivischer Ansicht,
- Fig. 6: weiteres Ausführungsbeispiel eines Wirbelsäulen-Implantats in perspektivischer Ansicht,
- Fig. 7: Wirbelsäulen-Implantat aus Fig. 6 in einer Ansicht von oben,
- Fig. 8: Wirbelsäulen-Implantat aus Fig. 6 in einer Ansicht von der Seite,
- Fig. 9: Wirbelsäulen-Implantat aus Fig. 6 in einer Ansicht von der bezüglich der Darstellung in Fig. 8 gegenüberliegenden Seite.

### Beschreibung des Ausführungsbeispiels

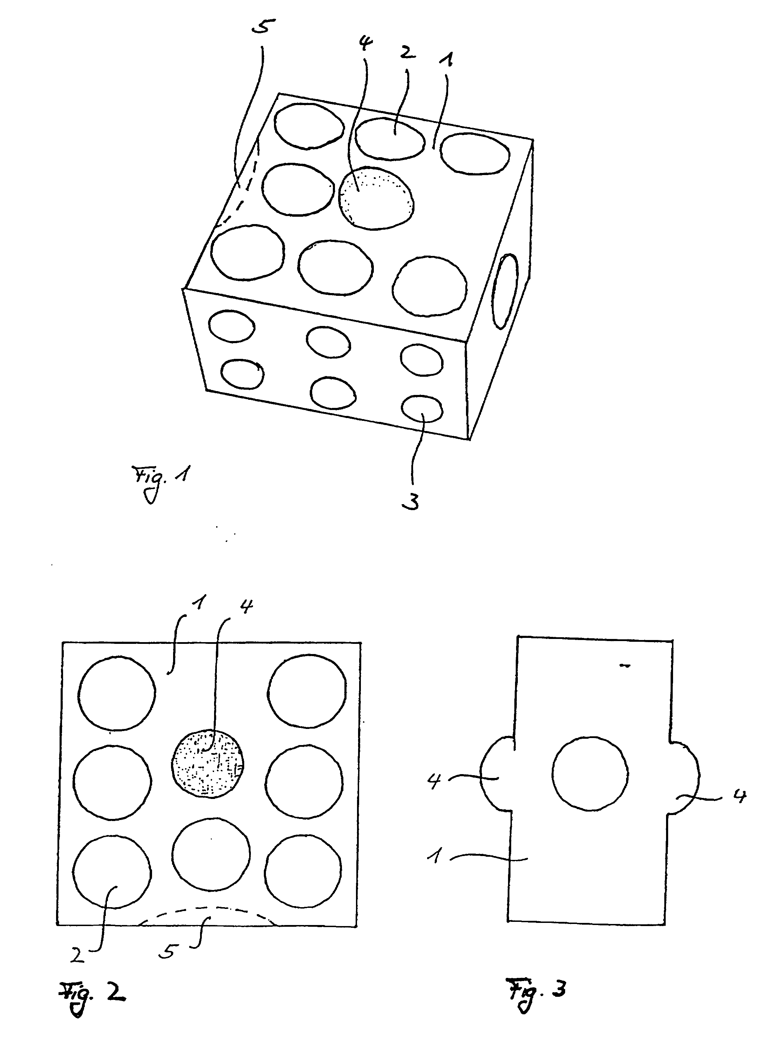

Fig. 1 zeigt ein Wirbelsäulen-Implantat mit einem flachen Körper 1, in dem durchgehende Kanäle 2 und 3 vorgesehen sind. Die Kanäle sind nach außen offen und verlaufen geradlinig im Inneren des Körpers. Sie verlaufen in zwei Richtungen. An dem flachen Körper 1 ist eine Erhebung 4 vorgesehen, die in eine Vertiefung des Wirbels eingreifen kann. Eine der Seiten des Wirbelsäulen-Implantats weist eine Einbuchtung 5 auf.

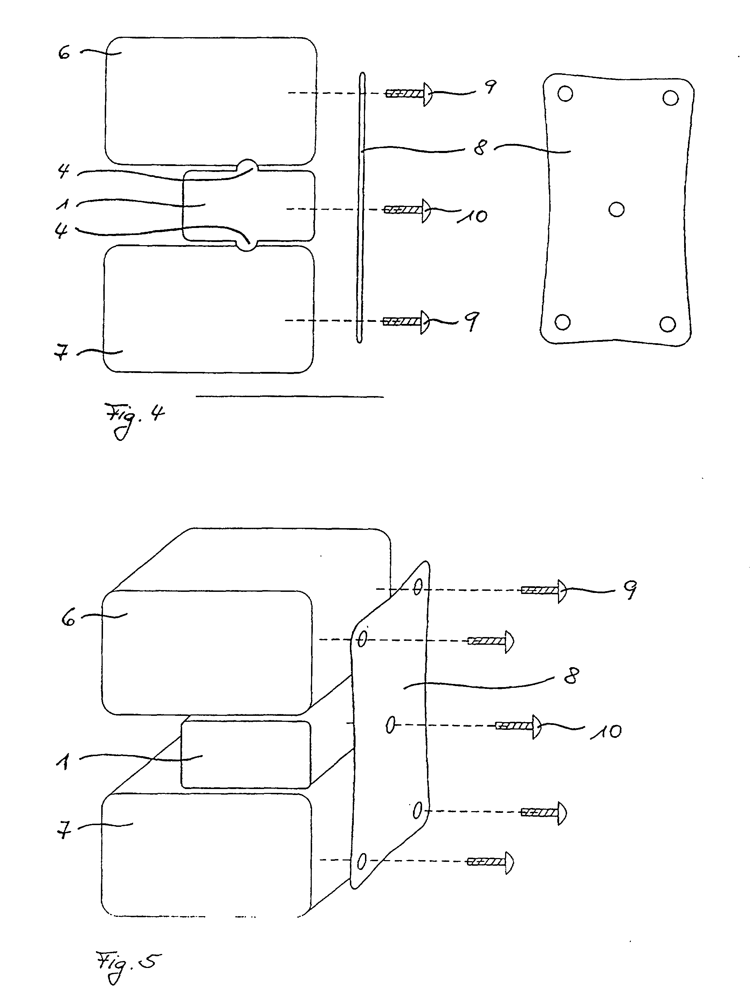

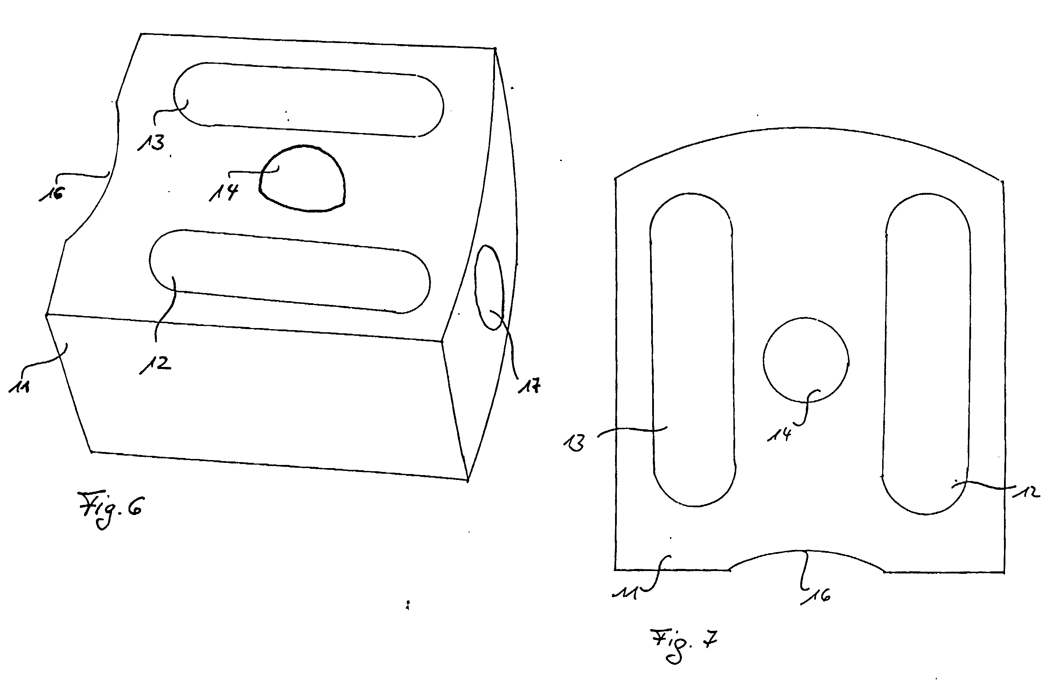

Die Fig. 2 und 3 zeigen das Wirbelsäulen-Implantat in verschiedenen Ansichten. In Fig. 3 ist erkennbar, daß die Erhebungen 4 auf gegenüberliegenden Seiten des flachen Körpers 1 angeordnet sind.

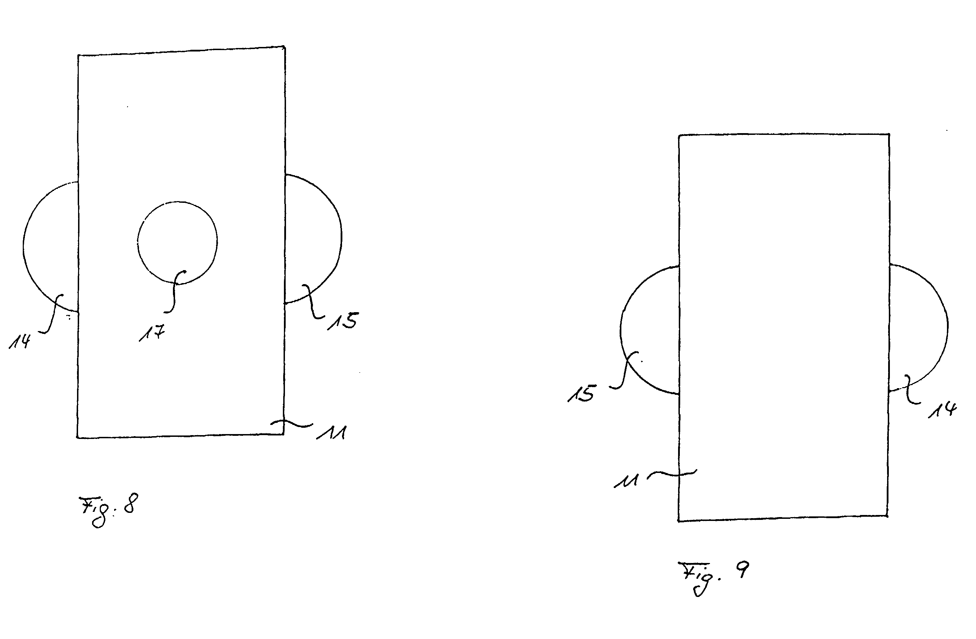

Fig. 4 und 5 zeigen schematisch das Wirbelsäulen-Implantat in dem zwischen zwei Wirbeln 6 und 7 eingesetzten Zustand. Die beiden Wirbel 6 und 7 sind dabei stark vereinfacht dargestellt. Die Erhebungen 4 greifen dabei in die Wirbel 6 und 7 ein. Zur zusätzlichen Stabilisierung und Stützung kann eine Platte 8 verwendet werden, die mit Hilfe von Knochenschrauben 9 an den Wirbeln und mit Hilfe einer Schraube 10 an dem Wirbelsäulen-Implantat befestigt wird.

In den Fig. 6 bis 9 ist ein weiteres Ausführungsbeispiel eines Wirbelsäulen-Implantats dargestellt. In einem flachen Körper 11 sind zwei Kanäle 12 und 13 mit länglichem Querschnitt, noppenartige Erhebungen 14 und 15, eine Einbuchtung 16 und eine Gewindebohrung 17 vorgesehen. Die Kanäle weisen ihre Öffnungen an den den Wirbeln im eingesetzten Zustand zugewandten Seiten auf. Sie können mit kleinen Knochteilen gefüllt werden. Hierbei erweist sich der relativ große Querschnitt der Kanäle als Vorteil. In die Gewindebohrung 17 kann eine Gewindestange zur Positionierung des Implantats zwischen den Wirbeln eingesetzt werden.

### Bezugszahlenliste

- 1: flacher Körper
- 2: Kanal
- 3: Kanal
- 4: Erhebung
- 5: Einbuchtung
- 6: Wirbel
- 7: Wirbel
- 8: Platte
- 9: Knochenschraube
- 10: Schraube
- 11: flacher Körper
- 12: Kanal
- 13: Kanal
- 14: noppenartige Erhebung
- 15: noppenartige Erhebung
- 16: Einbuchtung
- 17: Gewindebohrung

## Patentansprüche

1. Wirbelsäulen-Implantat zur Stabilisierung der Wirbelsäule, insbesondere der Halswirbelsäule
mit einem flachen, zwischen zwei Wirbel (6, 7) einsetzbaren Körper (1, 11), dessen Dicke ungefähr dem Abstand der Wirbel entspricht und
mit mehreren durchgängigen, nach außen offenen Kanälen (2, 3, 12, 13) in dem Körper (1, 11),
**dadurch gekennzeichnet,**
**daß** zum Verbinden des Wirbelsäulen-Implantats mit mindestens zwei Wirbeln (6, 7) an dem Körper (1, 11) an den im eingesetzten Zustand den Wirbeln zugewandten Seiten jeweils mindestens eine halbkugelförmige Erhebung (4, 14, 15) vorgesehen ist.

2. Wirbelsäulen-Implantat nach Anspruch 1, **dadurch gekennzeichnet, daß** als zusätzliche Befestigungsvorrichtung eine mit dem flachen Körper (1) und mit mindestens zwei Wirbeln (6, 7) verbindbare Platte (8) vorgesehen ist.

3. Wirbelsäulen-Implantat nach Anspruch 2, **dadurch gekennzeichnet, daß** in dem flachen Körper ein Gewinde zum Verbinden des Körpers mit der Platte vorgesehen ist.

4. Wirbelsäulen-Implantat nach einem der vorhergehenden Ansprüche, **dadurch gekennzeichnet, daß** die Öffnungen der Kanäle (12, 13) an den den Wirbeln zugewandten Seiten des Körpers (11) vorgesehen sind, daß die Kanäle (12, 13) einen länglichen Querschnitt aufweisen und mit kleinen Knochenteilen des behandelten Patienten befüllbar sind.

5. Wirbelsäulen-Implantat nach einem der vorhergehenden Ansprüche, **dadurch gekennzeichnet, daß** an dem Körper (11) an der im eingesetzten Zustand dem Nervenkanal der Wirbelsäule abgewandten Seite eine Gewindebohrung (17) zum Einsetzen eines Werkzeugs vorgesehen ist.

6. Wirbelsäulen-Implantat nach einem der vorhergehenden Ansprüche, **dadurch gekennzeichnet, daß** die den Wirbeln (6, 7) im eingesetzten Zustand zugewandte Fläche des Körpers (1) kleiner ist als die Ausdehnung der Wirbel in der zur Wirbelsäule senkrechten Ebene.

7. Wirbelsäulen-Implantat nach einem der vorhergehenden Ansprüche, **dadurch gekennzeichnet, daß** an dem Körper (1, 11) an der im eingesetzten Zustand dem Nervenkanal der Wirbelsäule zugewandten Seite eine Einbuchtung (5, 16) vorgesehen ist.

8. Wirbelsäulen-Implantat nach einem der vorhergehenden Ansprüche, **dadurch gekennzeichnet, daß** der Körper (1, 11) aus Polyethylen, insbesondere aus gepreßtem ultrahochmolekularem Niederdruckpolyethylen, besteht.

9. Wirbelsäulen-Implantat nach einem der Ansprüche 1 bis 7, **dadurch gekennzeichnet, daß** der Körper (1, 11) aus Titan besteht.

## Claims

1. Spinal implant for stabilising the vertebral column, in particular the cervical vertebral column,
with a flat body (1, 11) which is locatable between two vertebrae (6, 7) and
whose thickness corresponds approximately to the spacing of the vertebrae, and
with several continuous channels (2, 3, 12, 13) in the body (1, 11) which are open to the outside,
**characterised in that**
at least one semi-spherical round elevation (4, 14, 15) is provided to join the spinal implant with at least two vertebrae (6, 7) on the body (1, 11) on each side facing the vertebrae in the inserted condition.

2. Spinal implant according to claim 1, **characterised in that** a plate (8) joinable with the flat body (1) and with at least two vertebrae (6, 7) is provided as an additional fixing device.

3. Spinal implant according to claim 2, **characterised in that** a thread for joining the body with the plate is provided in the flat body.

4. Spinal implant according to one of the previous claims, **characterised in that** the openings of the channels (12, 13) are provided on the body (11) on the sides facing the vertebrae and that the channels (12, 13) have a long cross-section and can be filled with small bone parts of the treated patient.

5. Spinal implant according to one of the previous claims, **characterised in that** a threaded hole (17) for the insertion of a tool is provided on the body (11) on the side away from the nerve channel of the vertebral column in the inserted condition.

6. Spinal implant according to one of the previous claims, **characterised in that** the face of the body (1) facing the vertebrae (6, 7) in the inserted condition is smaller than the elongation of the vertebrae in the plane perpendicular to the vertebral column.

7. Spinal implant according to one of the previous claims, **characterised in that** an indentation (5, 16) is provided on the body (1, 11) on the side facing the nerve channel of the vertabral column in the inserted condition.

8. Spinal implant according to one of the previous claims, **characterised in that** the body (1, 11) consists of polyethylene, in particular of compressed ultra-high molecular low-pressure polyethylene.

9. Spinal implant according to one of claims 1 to 7, **characterised in that** the body (1, 11) consists of titanium.

## Revendications

1. Implant vertébral pour la stabilisation de la colonne vertébrale, en particulier de la colonne vertébrale cervicale
avec un corps (1, 11) plat, pouvant être inséré entre deux vertèbres (6,7), dont l'épaisseur correspond à peu près à l'espacement entre les vertèbres et
avec plusieurs canaux (2, 3, 12, 13) continus et ouverts vers l'extérieur dans le corps (1, 11),
**caractérisé,**
**en ce que**, pour la liaison de l'implant vertébral avec au moins deux vertèbres (6,7), il est prévu sur le corps (1, 11) au moins une éminence (4, 14, 15) ronde et hémisphérique sur chacun des côtés tournés vers les vertèbres dans l'état inséré.

2. Implant vertébral selon la revendication 1, **caractérisé en ce qu'**il est prévu comme dispositif de fixation supplémentaire une plaque (8) pouvant être reliée au corps (1) plat et à au moins deux vertèbres (6,7).

3. Implant vertébral selon la revendication 2, **caractérisé en ce qu'**il est prévu dans le corps plat un filetage pour la liaison du corps avec la plaque.

4. Implant vertébral selon l'une des revendications précédentes, **caractérisé en ce que** les ouvertures des canaux (12, 13) sont prévues sur les côtés du corps (11), tournés vers les vertèbres, **en ce que** les canaux (12, 13) présentent une section allongée et peuvent être remplis avec de petites parties d'os du patient traité.

5. Implant vertébral selon l'une des revendications précédentes, **caractérisé en ce qu'**il est prévu un alésage fileté (17) pour l'insertion d'un outil sur le corps (11) sur le côté opposé au canal du passage du nerf dans l'état inséré.

6. Spinal implant according to one of the previous daims, **characterised in that** the face of the body (1) facing the vertebrae (6, 7) in the inserted condition is smaller than the elongation of the vertebrae in the plane perpendicular to the vertebral column.

7. Spinal implant according to one of the previous daims, **characterised in that** an indentation (5, 16) is provided on the body (1, 11) on the side facing the nerve channel of the vertabral column in the inserted condition.

8. Spinal implant according to one of the previous daims, **characterised in that** the body (1, 11) consists of polyethylene, in particular of compressed ultra-high molecular low-pressure polyethylene.

9. Spinal implant according to one of claims 1 to 7, **characterised in that** the body (1, 11) consists oftitanium.
